# EUROPÄISCHE PATENTSCHRIFT

(11) **EP 1 103 283 B1**
(45) Veröffentlichungstag und Bekanntmachung des Hinweises auf die Patenterteilung: **25.10.2006**
(21) Anmeldenummer: 00250378.7
(22) Anmeldetag: 14.11.2000
(51) Int. Cl.: A61N 1/39

(54) **Implantierbarer Defibrillator**
Implantable defibrillator
Defibrillateur implantable

(30) Priorität: 23.11.1999 DE 19957481
(43) Veröffentlichungstag der Anmeldung: 30.05.2001
(73) Patentinhaber: BIOTRONIK GmbH & Co. KG, 12359 Berlin (DE)
(72) Erfinder: Schaldach, Max, 91054 Erlangen (DE); Poga, Sven, 48163 Münster (DE)
(74) Vertreter: Lindner-Vogt, Karin L.

(56) Entgegenhaltungen:
- EP-A- 0 362 611
- EP-A- 0 916 365
- US-A- 5 609 618
- US-A- 5 899 923

## Beschreibung

Die Erfindung betrifft einen implantierbaren Defibrillator mit einer aufladbaren Spannungsquelle von hoher Lebensdauer und mit einem Impulsgeber, welcher einen, bevorzugt über einen Spannungsvervielfacher, aufladbaren Kondensator aufweist.

Zur Aufladung des Kondensators wird der Spannungsquelle eine ausreichend große Energiemenge entnommen, um im Bedarfsfall durch den Impulsgeber über mit den Kondensatoranschlüssen verbundene Herzelektroden einen Defibrillationsstromstoß in das zu stimulierende Gewebe abgeben zu können. Die Energiemenge eines solchen bei Kammerflimmern zu applizierenden, impulsförmigen Gleichstromstoßes liegt im Bereich von 50 bis 500 Ws bei einer Impulsdauer im ms-Bereich.

Aus der europäischen Patentanmeldung EP 0 916 365 A1 und aus der amerikanischen Patentschrift US-PS 5 609 618 sind derartige Defibrillatoren bekannt, wobei der Spannungsvervielfacher als DC-DC-Stromwandler ausgebildet ist.

Um eine sichere Betriebsführung für einen Defibrillator zu erreichen, sind Sensormittel vorgesehen, um bereits beim Eintreten von erfahrungsgemäß als Vorzeichen für ein baldiges Herzflimmern zu wertenden Gewebezuständen die Aufladung des Kondensators des Impulsgebers einleiten zu können, damit der Kondensator im Bedarfsfall bereits die für den Gleichstromstoß erforderliche Energiemenge aufgenommen hat.

Für den Fall, daß das Herzflimmern ausbleibt, muß diese Energiemenge bei nicht aktiviertem Defibrillator entsprechend geltender Sicherheitsvorschriften - wie in EP 0 916 365 A1 erläutert - intern abgebaut werden. Dadurch entstehende Energieverluste führen in nachteiliger Weise zu einer vorzeitigen Herabsetzung der Leistungsfähigkeit der Batterie, so daß ein Austausch des Defibrillators durch Reimplantation zu einem entsprechend früheren Zeitpunkt erfolgen muß.

Aus EP 0 362 611 ist bekannt, daß eine Rückführungsleitung vorgesehen werden kann, die eine Rückführungnichtverbrauchter oder nicht zur Gewebestimulation benötigter elektrischer Energie vom Kondensator zur Spannungsquelle erlaubt.

Ausgehend von den Mängeln des Standes der Technik liegt der Erfindung deshalb die Aufgabe zugrunde, einen Defibrillator der eingangs genannten Gattung mit einem verbesserten Schaltungsaufbau anzugeben, durch welchen ein besonders energieökonomischer Betrieb des Defibrillators möglich ist, um durch Erhöhung der Lebensdauer der Spannungsquelle eine Verlängerung des Wechselzyklus des Defibrillators zu erreichen.

Diese Aufgabe wird erfüllungsgemäß durch einen Defibrillator der eingangs genannten Art gelöst, mit einer Rückführungsleitung zwischen dem Kondensator und der Spannungsquelle, die so gestaltet ist, daß über die Rückführungsleitung nicht verbrauchte oder nicht zur Gewebestimulation benötigte elektrische Energie vom Kondensator zur Spannungsquelle rückführbar ist.

Die Erfindung schließt die Erkenntnis ein, daß in eine wiederaufladbare Batterie Energie eingespeist werden kann, wenn an die Batterie eine Spannung angelegt wird, welche einen größeren Wert aufweist, als die momentan an der Batterie vorhandene Klemmenspannung. Somit ist es beispielsweise möglich, Energie in Form von in einem Kondensator gespeicherter elektrischer Ladung zu einer Batterie zurückzuführen, wenn die Kondensatorspannung die Klemmenspannung der Batterie übersteigt.

Erfindugsgemäß ist bei einem zur Stimulation von menschlichem Herzgewebe implantierten Defibrillator eine Rückführungsleitung zwischen dem Kondensator und der als aufladbare Batterie ausgebildeten Spannungsquelle vorgesehen, über welche eine Rückführung von nicht verbrauchter bzw. nicht zur Gewebestimulation benötigter elektrischer Energie von dem Kondensator zu Batterie erfolgt. Dadurch kann in vorteilhafter Weise die Leistungsfähigkeit der Batterie verbessert und ihre Lebensdauer verlängert werden, so daß ein Austausch des Defibrillators durch eine Reimplantation aufgrund einer verbrauchten Batterie erst zu einem späteren Zeitpunkt vorgenommen werden muß, was für den betreffenden Patienten besonders günstig ist.

In der Rückführungsleitung ist eine Schaltvorrichtung angeordnet, um die Energierückführung von dem auf die für das Defibrillieren erforderliche Hochspannung aufgeladenen Kondensator zur Batterie auf einfache Weise zeitlich steuern zu können. Eine derartige Steuerungsmöglichkeit ist erforderlich, da durch die Rückführungsleitung die Betriebsführung des Defibrillators nicht beeinträchtigt werden und der Energierückfluß nur dann erfolgen darf, wenn mit Sicherheit feststeht, daß eine Gewebestimulation nicht mehr notwendig ist.

Entsprechend der Lehre der Erfindung ist die in der Rückführungsleitung angeordnete Schaltvorrichtung durch einen Taktgenerator aktivierbar ausgebildet. Der Taktgenerator erzeugt bei Ansteuerung eine impulsförmige Ausgangsspannung, wobei durch die Impulse ein Zeitfenster ist derart gewählt, daß es mit Sicherheit einen Zeitbereich erfaßt, der sich mindestens von dem Zeitpunkt, an welchem durch Sensormittel ein Zustand des Gewebes detektiert wird, bei welchem - entsprechend Erfahrungswerten - mit größter Wahrscheinlichkeit kurzfristig eine Gewebestimulation erforderlich wird, bis zu dem Zeitpunkt erstreckt, wo die Gewebestimulation - falls erforderlich - abschlossen worden ist.

Ist der zur Gewebestimulation vorgesehene Zeitpunkt überschritten und keine Gewebestimulation ausgelöst worden, so erfolgt eine Aktivierung der Schaltvorrichtung in der Rückführungsleitung. Die Verbindung zwischen Kondensator und Batterie wird hergestellt und die nicht verbrauchte Energiemenge wird in die Batterie eingespeist.

Für die Koordinierung des Aktivierens des die Schaltvorrichtung in der Rückführungsleitung steuernden Taktgenerators und des Einschaltens des Impulsgebers zur Erzeugung der für eine gegebenenfalls durchzuführende Gewebestimulation erforderliche Kondensatorladung ist eine separate Steuerung vorgesehen. Der Signaleingang dieser Steuerung ist mit in dem zu stimulierenden Gewebe positionierten Sensormitteln verbunden, durch welche der Zustand des Gewebes ständig überwacht wird. Die Signalausgänge der Steuerung sind mit dem Impulsgeber und mit dem Taktgenerator verbunden, so daß bei entsprechende Anzeichen für eine kurzfristig notwendig werdende Gewebestimulation aufweisenden Sensorsignalen gleichzeitig eine Aktivierung des Impulsgebers und des Taktgenerators erfolgt. Der Taktgenerator aktiviert seinerseits die Schalteinrichtung in der Energierückführungsleitung, wobei durch den ersten, das Zeitfenster des Taktgenerators begrenzenden Impuls die Energierückführungsleitung unterbrochen wird, so daß die Aufladung des Kondensators in dem Impulsgebers erfolgen kann.

Erfolgt nach einer durch den zweiten Impuls des Zeitfensters des Taktgebers bestimmten Zeit keine Gewebestimulation, beispielsweise, weil sich das Herzgewebe selbst stabilisiert hat, so wird die Schalteinrichtung in der Energierückführungsleitung durch den zweiten Impuls des Zeitfensters erneut aktiviert und stellt die Leitungsverbindung zwischen Batterie und Kondensatoranschluß wieder her.

Entsprechend einer vorteilhaften Weiterbildung der Erfindung ist in der den Impulsgenerator mit dem Signalausgang der Steuerung verbindenden Steuerleitung ein Zeitverzögerungsglied vorgesehen, um auf einfache Weise zu erreichen, daß die Energierückführungsleitung mit Sicherheit unterbrochen ist, bevor das Aufladen des Kondensators auf ein für den Gleichstromstoß erforderliche Spannungsniveau beginnt.

Nach einer günstigen Variante der Erfindung ist für das Zeitfenster, welches mindestens einen Bereich vom Zeitpunkt des Einschaltens des Impulsgenerators bis zum theoretischen Zeitpunkt des Auslösens einer erforderlichen Gewebestimulation umfaßt, ein Sicherheitszeitbereich vorgesehen, welcher einen Wert von wenigstens 10% des mindestens erforderlichen Zeitfensters aufweist.

In einer anderen vorteilhaften Variante der Erfindung ist vorgesehen, eine telemetrisch programmierbaren Taktgenerator einzusetzen, um in bequemer Weise eine patientenspezifische Einstellung des Zeitfensters vornehmen zu können

Andere vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt.

Es zeigen:
- Figur 1: eine bevorzugte Ausführungsform der Erfindung in Form eines Blockschaltbildes,sowie
- Figur 2: das Impulsdiagramm eines im Blockschaltbild gemäß Figur 1 gezeigten Taktgenerators.

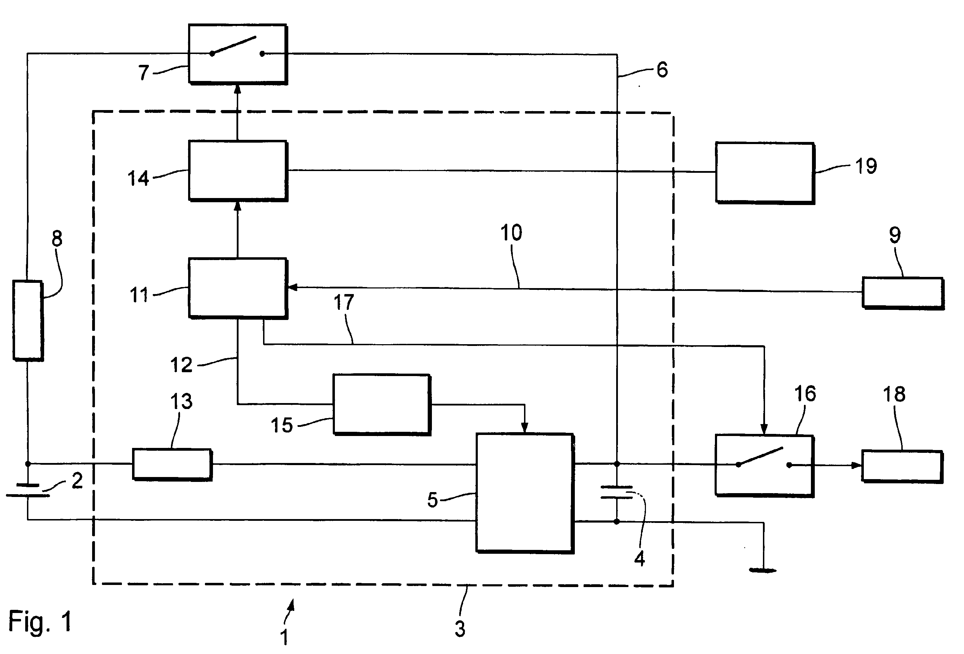

Der implantierbare Defibrillator 1, dessen Blockschaltbild in Figur 1 dargestellt ist, weist einen durch eine wiederaufladbare Batterie 2 gespeisten Impulsgeber 3 auf.

Der Kondensator 4 des Impulsgebers 3 wird von der Batterie 2 über einen Spannungsvervielfacher 5 auf eine hohe Spannung aufgeladen, um im Bedarfsfall einen Spannungsstoß zum Stimulieren von Herzgewebe abgeben zu können.

Der Kondensator 4 ist durch eine Energierückführungsleitung 6, in welcher eine Schalter 7 und ein Vorwiderstand 8 angeordnet sind, mit dem Pluspol der Batterie 2 verbunden. Bei aufgeladenem Kondensator 4 kann die gespeicherte Energie in dem Fall, daß eine Stimulation des Herzgewebes überflüssig ist, durch Schließen des Schalter zu der Batterie 2 zurückgeführt werden.

Der Zustand des gegebenenfalls zu stimulierenden Herzgewebes wird durch Elektroden 9 detektiert, welche über eine Elektrodenleitung 10 mit einem elektronischen Steuerbaustein 11 verbunden sind. Der Steuerbaustein 11 aktiviert über seine Ausgangsleitung 12 den über einen Vorwiderstand 13 mit der Batterie 2 verbundenen Spannungsvervielfacher 5, wenn sein Signaleingang über die Elektrodenleitung 10 durch ein Signal angesteuert wird, welches immer dann detektiert wird, wenn sich das Herzgewebe in einem Zustand befindet, bei dem in relativ kurzer Zeit ein Kammerflimmern zu erwarten ist und eine Defibrillation durch einen Gleichspannungsstoß mit sehr großer Wahrscheinlichkeit erforderlich sein wird.

Um zu sichern, daß der Kondensator 4 des Impulsgebers 3 auf die zum Defillibrieren erforderliche Spannungsniveau geladen werden kann, wird durch den Steuerbaustein 1 1 gleichzeitig ein Taktgeber 14 aktiviert, an dessen Signalausgang eine Impulsspannung in Form eines durch zwei Spannungsimpulse begrenztes Zeitfensters zur Verfügung steht. Der erste Spannungsimpuls des Zeitfensters öffnet den Schalter 7 in der Energierückführungsleitung 6, um die direkte Verbindung zwischen Batterie und Kondensator zu unterbrechen.

Durch ein Verzögerungsglied 15 in der Ausgangsleitung 12 des Steuerungsbausteins 11 wird erreicht, daß der Spannungsvervielfacher zur Aufladung des Kondensators 4 erst dann eingeschaltet wird, nachdem die Rückführungsleitung 6 unterbrochen worden ist.

Wird durch die Sensorelektroden ein ein Defillibrieren erfordernder Zustand des Herzgewebes detektiert, erfolgt durch den Defillibrator 1 die Abgabe eines Gleichstromstoßes nach Aktivieren eines Schalters 16 durch ein Schaltsignal auf der Ausgangsleitung 17 des Steuerbausteins 11. Die Defibrillationselektrode ist mit 18 bezeichnet.

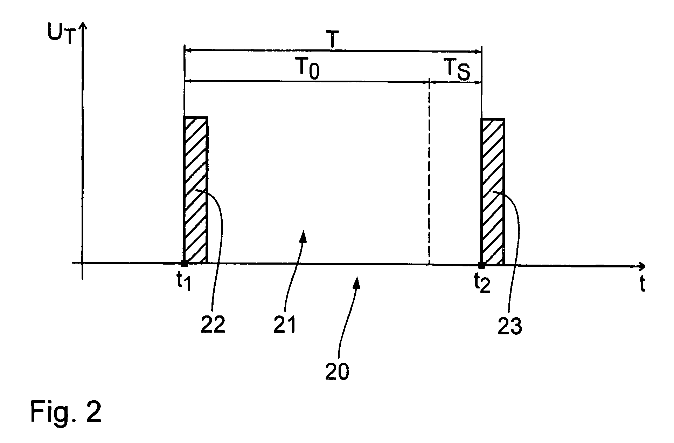

Da für den Zeitraum für das Auftreten der ersten Anzeichen eines möglichen Kammerflimmern des Herzen und dem Eintreten des Kammerflimmerns gesicherte Erfahrungswerte zur Verfügung stehen, wird für die Breite des von dem Taktgenerator 14 erzeugten Zeitfensters (vergleiche die Positionsziffer 21 in Figur 2) ein Wert gewählt, welcher mindestens der Zeit vom Zeitpunkt des Detektieren von ersten Vorzeichen eines zu erwartenden Kammerflimmerns bis zum Zeitpunkt des tatsächlichen Eintretens eines Kammerflimmers entspricht. Die Größe des durch den Taktgeber 14 erzeugbaren Zeitfensters ist einstellbar und wird bei implantiertem Defibrillator durch eine Telemetrieeinrichtung 19 programmiert.

Der von dem Taktgenerator 14 abgegebene zweite, das Ende des Zeitfensters begrenzende Spannungsimpuls schließt den Schalter 7 in der Energierückführungsleitung 6 und die nicht zum Defillibrieren benötigte Energie fließt unter Entladung des Kondensators 4 zur Batterie 2 zurück.

Das in Figur 2 dargestellte Diagramm 20 zeigt den zeitlichen Verlauf der Ausgangsspannung U_{T} des in Figur 1 gezeigten Taktgenerators 14. Die Länge T = t₁ - t₂ des Zeitfensters 21 wird durch die vorderen Flanken der von dem Taktgeber erzeugten Impulse 22 und 23 bestimmt. Aus Sicherheitsgründen ist in dem Zeitfenster 21 ein Zeitbereich T_{S} vorgesehen. Die Verlängerung des durch den Zeitpunkt zwischen Detektion der ersten Anzeichen eines möglichen Eintritts von Kammerflimmern und dem Zeitpunkt der Detektion des wirklich aufgetretenen Flimmern der Herzkammern bestimmten Zeitbereichs T_{O} des Zeitfensters 21 gleicht mögliche patientenspezifische Toleranzen dieses Zeitbereichs aus. Dies sichert, daß die Rückführung von in dem Kondensator des Impulsgebers gespeicherten Energie bzw. eine Restenergiemenge zur Batterie (vergleiche die Positionsziffern 2, 3 und 4 in Figur 1) erst dann erfolgt, wenn ein Kammerflimmern ausgeblieben ist und in Kürze nicht mehr eintreten wird bzw. eine Stimulation des Herzgewebes aufgrund von Kammerflimmern vorgenommen wurde.

Da der Taktgenerator 14 telemetrisch programmierbar ausgebildet ist, können die medizinischen Erfahrungswerte der Zeit zwischen Detektieren der ersten Anzeichen eines möglichen Kammerflimmerns und dem Zeitpunkt des tatsächlichen Eintretens des Kammerflimmerns mit patientenspezifischen Besonderheiten ohne besondere Mühe zur Festlegung des optimalen Zeitfensters des Taktgenerators berücksichtigt werden.

## Patentansprüche

1. Implantierbarer Defibrillator (1) mit
- einer aufladbaren Spannungsquelle (2),
- einem Impulsgeber (3), welcher aus einem Spannungsvervielfacher (5) und einem auf die Defibrillationsspannung aufladbaren Kondensator (4) gebildet ist,
- einer Rückführungsleitung (6) zwischen dem Kondensator (4) und der Spannungsquelle (2), die durch eine Schaltvorrichtung (7) auftrennbar ausgebildet und so gestaltet ist, dass über die Rückführungsleitung (6) nichtverbrauchte oder nicht zur Gewebestimulation benötigte elektrische Energie vom Kondensator (4) zur Spannungsquelle(2) rückführbar ist, wobei der Impulsgeber (3) zur Aktivierung der Schaltvorrichtung (7) einen Taktgenerator (14) aufweist,
**dadurch gekennzeichnet,**
**dass** der Taktgenerator (14) ausgebildet ist, ein Zeitfenster (21) zu erzeugen, nach dessen Ablauf durch die Schaltvorrichtung (7) eine Verbindung (6) zwischen Kondensator (4) und Spannungsquelle (2) hergestellt wird.

2. Defibrillator nach Anspruch 1, **gekennzeichnet durch** eine mit in dem zu stimulierenden Gewebe angeordneten Sensormitteln (9) verbundene Steuerung (11), **durch** welche der Spannungsvervielfacher (5) und der Taktgenerator (14) des Impulsgebers (3) jeweils dann aktiviert werden, wenn die Sensormittel (9) einen Zustand des Gewebes detektieren, welcher kurzfristig eine Gewebestimulation erfordert.

3. Defibrillator nach Anspruch 1 oder Anspruch 2, **dadurch gekennzeichnet, dass** das Zeitfenster (21) größer ist als der Zeitbereich vom Zeitpunkt des Aktivierens des Spannungsvervielfachers (5) des Impulsgebers (3) bis zum theoretischen Zeitpunkt des Auslösens einer erforderlichen Gewebestimulation.

4. Defibrillator nach Anspruch 3, **dadurch gekennzeichnet, dass** für das Zeitfenster (21) ein Sicherheitszeitbereich (Tₛ) vorgesehen ist, welcher einen Wert ≥10% des mindestens erforderlichen Zeitfensters aufweist.

5. Defibrillator nach einem der vorhergehenden Ansprüche, **gekennzeichnet durch** ein Zeitverzögerungsglied (15), welches in der den Signalausgang der Steuerung (11) mit dem Spannungsvervielfacher (5) des Impulsgebers (3) verbindenden Steuerleitung (12) angeordnet ist.

6. Defibrillator nach einem der vorhergehenden Ansprüche, **dadurch gekennzeichnet, dass** der Taktgenerator (14) telemetrisch programmierbar ausgebildet ist.

## Claims

1. An implantable defibrillator (1) having
- a chargeable voltage source (2),
- a pulse generator (3), which is formed by a voltage multiplier (5) and a capacitor (4), which is chargeable to the defibrillation voltage,
- a return line (6) between the capacitor (4) and the voltage source (2), which is implemented as disconnectable by a switching device (7) and is designed in such a way that unused electrical energy or electrical energy which is not required for tissue stimulation is returnable via the return line (6) from the capacitor (4) to the voltage source (2), the pulse generator (3) having a clock generator (14) to activate the switching device (7),
**characterized in that** the clock generator (14) is implemented to generate a time window (21), after whose expiration a connection (6) is produced between capacitor (4) and voltage source (2) by the switching device (7).

2. The defibrillator according to claim 1, **characterized by** a controller (11), connected to sensor means (9) situated in the tissue to be stimulated, through which the voltage multiplier (5) and the clock generator (14) of the pulse generator (3) are each activated when the sensor means (9) detect a state of the tissue which imminently requires tissue stimulation.

3. The defibrillator according to claim 1 or claim 2, **characterized in that** the time window (21) is longer than the time range from the point in time of the activation of the voltage multiplier (5) of the pulse generator (3) up to the theoretical point in time of the triggering of a required tissue stimulation.

4. The defibrillator according to claim 3, **characterized in that** a safety time range (T_{S}), which has a value ≥ 10% of the minimum required time window, is provided for the time window (21).

5. The defibrillator according to one of the preceding claims, **characterized by** a time delay element (15), which is situated in the control line (12) connecting the signal output of the controller (11) to the voltage multiplier (5) of the pulse generator (3).

6. The defibrillator according to one of the preceding claims, **characterized in that** the clock generator (14) is implemented so it is programmable through telemetry.

## Revendications

1. Défibrillateur (1) implantable comprenant
- une source de tension (2) rechargeable,
- un générateur d'impulsions (3), qui est formé d'un multiplicateur de tension (5) et d'un condensateur (4) rechargeable jusqu'à la tension de défibrillation
- une ligne de recyclage (6) entre le condensateur (4) et la source de tension (2), qui est réalisée de façon séparable par un dispositif de commutation (7) et est conçue de telle sorte que l'énergie électrique non utilisée par la ligne de recyclage (6) et non utilisée pour la stimulation de tissu peut être recyclée depuis le condensateur (4) vers la source de tension (2), le générateur d'impulsions (3) pour l'activation du dispositif de commutation (7) présentant une horloge interne (14),
**caractérisé en ce que**
l'horloge interne (14) est conçue pour générer une fenêtre de temps (21), après l'écoulement de laquelle une liaison (6) entre le condensateur (4) et la source de tension (2) est établie par le dispositif de commutation (7).

2. Défibrillateur selon la revendication 1, **caractérisé par** une commande (11) reliée aux moyens de détection (9) disposés dans le tissu à stimuler, par laquelle le multiplicateur de tension (5) et l'horloge interne (14) du générateur d'impulsions (3) sont activés à chaque fois dans les cas où les moyens de détection (9) détectent un état du tissu qui exige à court terme une stimulation de tissu.

3. Défibrillateur selon la revendication 1 ou 2, **caractérisé en ce que** la fenêtre de temps (21) est plus grande que la plage de temps s'écoulant depuis le moment de l'activation du multiplicateur de tension (5) du générateur d'impulsions (3) jusqu'au moment théorique du déclenchement d'une stimulation de tissu nécessaire.

4. Défibrillateur selon la revendication 3, **caractérisé en ce qu'**il est prévu pour la fenêtre de temps (21) une plage de temps de sécurité (Tₛ) qui présente une valeur supérieure à 10 % de la fenêtre de temps au moins nécessaire.

5. Défibrillateur selon l'une quelconque des revendications précédentes, **caractérisé par** un élément de temporisation (15), qui est disposé dans la ligne de commande (12) reliant la sortie de signal de la commande (11) au multiplicateur de tension (5) du générateur d'impulsions (3).

6. Défibrillateur selon l'une quelconque des revendications précédentes, **caractérisé en ce que** l'horloge interne (14) est conçue de façon programmable par télémétrie.
